(19) Europäisches Patentamt / European Patent Office / Office européen des brevets

(11) **EP 1 827 586 B1**

(12) **EUROPÄISCHE PATENTSCHRIFT**

(45) Veröffentlichungstag und Bekanntmachung des Hinweises auf die Patenterteilung:
**20.02.2013 Patentblatt 2013/08**

(21) Anmeldenummer: **05848369.4**

(22) Anmeldetag: **06.12.2005**

(51) Int Cl.:
**A61N 1/32** (2006.01)     **A61N 1/05** (2006.01)

(86) Internationale Anmeldenummer:
**PCT/DE2005/002192**

(87) Internationale Veröffentlichungsnummer:
**WO 2006/063558 (22.06.2006 Gazette 2006/25)**

(54) **VORRICHTUNG ZUR DESYNCHRONISATION NEURONALER HIRNAKTIVITÄT**

DEVICE FOR DESYNCHRONIZING NEURONAL BRAIN ACTIVITY

DISPOSITIF DE DESYNCHRONISATION DE L'ACTIVITE CEREBRALE NEURONALE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **16.12.2004 DE 102004060514**

(43) Veröffentlichungstag der Anmeldung:
**05.09.2007 Patentblatt 2007/36**

(73) Patentinhaber: **Forschungszentrum Jülich GmbH 52425 Jülich (DE)**

(72) Erfinder: **TASS, Peter 81541 München (DE)**

(74) Vertreter: **Manitz, Finsterwald & Partner GbR Martin-Greif-Strasse 1 80336 München (DE)**

(56) Entgegenhaltungen:

| | |
|---|---|
| WO-A-03/077986 | US-A- 4 338 945 |
| US-A- 4 541 432 | US-A- 6 038 480 |
| US-A- 6 094 598 | US-A1- 2002 099 418 |
| US-A1- 2003 135 248 | US-B1- 6 535 767 |
| US-B1- 6 671 557 | |

**Beschreibung**

Die Erfindung betrifft eine Vorrichtung zur Desynchronisation neuronaler Hirnaktivität.

Bei Patienten mit neurologischen oder psychiatrischen Erkrankungen wie beispielsweise Morbus Parkinson, essentiellem Tremor, Dystonie oder Zwangserkrankungen sind Nervenzellenverbände in umschriebenen Bereichen des Gehirns, z. B. des Thalamus und der Basalganglien, krankhaft aktiv, zum Beispiel übersteigert synchron. In diesem Falle bildet eine große Anzahl von Neuronen synchron Aktionspotentiale aus, das heißt die beteiligten Neuronen feuern übermäßig synchron. Beim gesunden Patienten feuern die Neuronen in diesen Hirngebieten qualitativ anders, zum Beispiel auf unkorrelierte Weise.

Beim Morbus Parkinson verändert die pathologisch synchrone Aktivität die neuronale Aktivität in Arealen der Großhirnrinde, wie zum Beispiel im primären motorischen Cortex, indem sie diesen beispielsweise ihren Rhythmus aufzwingt, so dass schließlich die von diesen Arealen gesteuerten Muskeln pathologische Aktivität, z. B. ein rhythmisches Zittern, entfalten.

Die Schrift WO 2004/093981 A1 beschreibt die Stimulation von Neuronenpopulationen mit N Elektroden, wobei unterschiedliche Elektroden zeitversetzt um ein N-tel der pathologischen Periode Hochfrequenz-Pulszüge applizieren.

Es ist die Aufgabe der vorliegenden Erfindung, eine Vorrichtung zu schaffen, mit denen Patienten behandelt werden können, ohne dass dabei eine Adaptation an einen unphysiologischen Dauerreiz stattfindet, bei denen keine langwierige Kalibrierung notwendig ist und zwar auch dann nicht, wenn die Hauptfrequenz-Komponente der pathologisch rhythmischen Aktivität starken Schwankungen unterliegt. Nebenwirkungen sollen vermindert oder sogar unterbunden werden. Weiterhin soll die Notwendigkeit eines harten Phasenresets entfallen, das heißt, der Ladungseintrag soll minimiert werden. Weiterhin soll die Wirksamkeit so verbessert werden, dass auch Patienten mit schwersten Krankheitsbildern erfolgreich behandelt werden können.

Ausgehend vom Oberbegriff des Anspruchs 1 wir die Aufgabe erfindungsgemäß gelöst mit den im kennzeichnenden Teil des Anspruchs 1 angegebene Merkmalen.

Mit der erfindungsgemäßen Vorrichtung ist es nunmehr möglich, die Symptome der Patienten zu lindern oder völlig zu unterdrücken. Es können auch Patienten mit schwersten Krankheitsbildern erfolgreich behandelt werden. Es können sogar kurative Effekte erzielt werden, d. h. es werden nicht nur einfach unter Stimulation die Symptome unterdrückt, sondern es findet eine Heilung in den Neuronen statt, so dass stimuliertes Gewebe die krankhafte Neigung zur synchronen Aktivität verlernt.

Die Zeichnungen zeigen eine für die Durchführung des Verfahrens geeignete Vorrichtung.

Es zeigt:

Fig.1: Eine erfindungsgemäße Vorrichtung

Fig.2a: Neuronale Feuermuster bei Stimulation mit fünf Abfolgen von Sequenzen über drei Stimulationskontakte

Fig.2b: Zu 2a zugehöriges lokales Feldpotential der stimulierten Neuronenpopulationen

Fig.3: Mehrkontaktelektrode

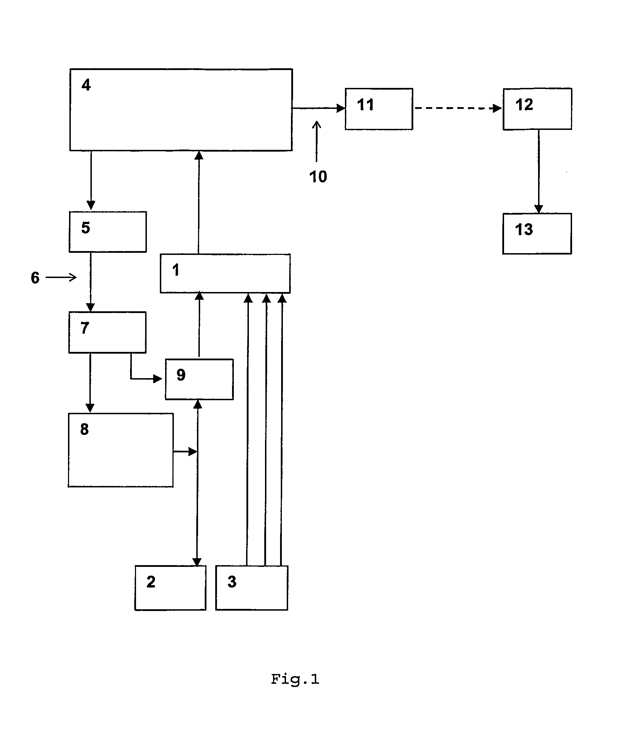

Die Vorrichtung gemäß Figur 1 umfasst einen Trennverstärker 1, an den mindestens eine Elektrode 2 sowie Sensoren 3 zur Erfassung von physiologischen Messsignalen angeschlossen sind. Im Fall des Vorliegens einer Elektrode 2 umfasst diese mindestens zwei Kotaktstellen, welche Reize angeben können. Beim Vorliegen von mehr als einer Elektrode müssen mindestens zwei Elektroden jeweils mindestens eine Kontaktstelle besitzen, welche elektrische Reize abgeben kann. Der Trennverstärker steht weiterhin mit einer Einheit 4 zur Signalverarbeitung und Steuerung in Verbindung, welche an einen optischen Sender für die Stimulation 5 angeschlossen ist. Der optische Sender 5 ist über Lichtwellenleiter 6 mit einem optischen Empfänger 7 verbunden, welcher mit einer Stimulatoreinheit 8 zur Signalerzeugung in Verbindung steht. Die Stimulatoreinheit 8 für die Signalerzeugung steht mit mindestens einer Elektrode 2 in Verbindung. Am Eingangsbereich der Elektroden 2 in den Trennverstärker 1 befindet sich ein Relais 9 oder Transistor. Die Einheit 4 steht über eine Leitung 10 mit einem Telemetriesender 11 in Verbindung, welcher mit einem Telemetrieempfänger 12 in Verbindung steht, der sich außerhalb des zu implantierenden Geräts befindet und an den ein Mittel zur Visualisierung, Verarbeitung und Speicherung der Daten 13 angeschlossen ist.

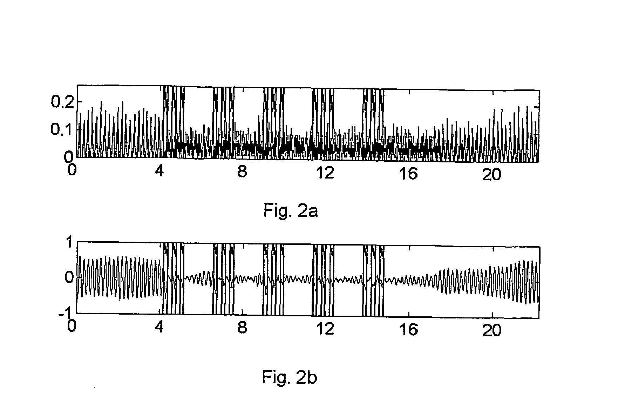

Die Figur 2a zeigt das Summenaktionspotential einer krankhaft aktiven Neuronenpopulation, das heißt, die relative Anzahl der von den Neuronen produzierten Aktionspotentiale (das heißt, die Anzahl der von den Neuronen produzierten Aktionspotentiale dividiert durch die Anzahl aller Neuronen der gesamten Neuronenpopulation) vor, während und nach einer Stimulation. Letztere besteht aus fünf durch vier gleich lange Pausen getrennte identische Reihenfolgen von Sequenzen. Jede Reihenfolge von Sequenzen besteht aus einer niederfrequenten Abfolge von drei Sequenzen. Der Beginn und das Ende einer Sequenz sind jeweils durch einen vertikalen Strich gekennzeichnet. Jede Sequenz hat dieselbe Konfiguration, das heißt, es wird jedes Mal in der gleichen Reihenfolge, durch kurze Pausen voneinander getrennt, jeweils ein Hochfrequenz-Pulszug sequentiell über jeweils drei Kontaktstellen appliziert. Für die drei Kontaktstellen wird jeweils derselbe Hochfrequenz-Pulszug verwendet. Die Pausen zwischen den drei Hochfrequenz-Pulszügen einer Sequenz entsprechen ungefähr einem Drittel der mittleren Periode der zu desynchronisierenden neuronalen Aktivität. Die Abszisse ist die Zeitachse in Sekunden, und die Ordinate ist dimensionslos.

Figur 2b zeigt das zu dem in Figur 2a gezeigten Stimulationsvorgang gehörende lokale Feldpotential der stimulierten krankhaft aktiven Neuronenpopulation. Die Abszisse ist die Zeitachse in Sekunden, und die Ordinate zeigt das lokale Feldpotential in auf das Maximum normierten willkürlichen Einheiten.

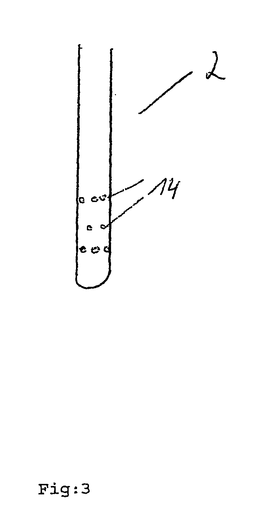

Figur 3 zeigt eine Mehrkontaktelektrode 2 mit den Kontaktstellen 14.

Als Sensoren 3 können beispielsweise epikortikale Elektroden, Tiefenelektroden, Hirnelektroden oder periphere Elektroden eingesetzt werden.

Bei der Elektrode 2 handelt es sich um mindestens zwei Drähte, an deren Enden eine Potentialdifferenz zum Zwecke der Stimulation angelegt wird. Die Enden, an denen eine Potentialdifferenz angelegt wird, stellen Kontaktstellen 14 dar, welche mit dem zu stimulierenden Gewebe in Kontakt treten, um sie mit elektrischen Reizen zu stimulieren. Sie werden im Folgenden als Kontaktstellen 14 bezeichnet. Eine Elektrode kann über mindestens eine Kontaktstelle 14 verfügen. Im Folgenden wird die Erfindung auf Kontaktstellen 14 bezogen. Dabei soll die Zahl der Kontaktstellen 14 nicht zwingend mit der Zahl der Elektroden übereinstimmen, vielmehr können n+m Kontaktstellen 14 auf n Elektroden verteilt sein. m ist dabei vorzugsweise größer als n. Die Elektrode 2 ist ein Mittel zur Reizapplikation.

Im weiteren Sinne kann sie auch ein Mittel zur Messung von physiologischen Signalen sein. Zur Messung der physiologischen Signale können grundsätzlich die gleichen Kontaktstellen 14 verwendet werden, die zur Stimulation herangezogen werden.

Als Kontaktstelle 14, die in der Fachwelt als "stimulation site" bezeichnet wird, wird daher im Folgenden eine gewebsseitige Drahtfläche bezeichnet, welche geeignet ist einen elektrischen Reiz zu applizieren und/oder ein physiologisches Signal zu messen.

Es kann sich dabei um Makro- oder Mikroelektroden handeln.

Zusätzlich aber nicht zwingend kann über die Elektrode 2 eine Potentialdifferenz gemessen werden, um eine pathologische Aktivität festzustellen.

In einer weiteren Ausführungsform kann die Elektrode 2 auch nur aus einem einzelnen Draht bestehen. In diesem Falle wird zum Zwecke der Stimulation eine Potentialdifferenz zwischen dem Ende dieses Drahtes einerseits und einem metallischen Gegenstück andererseits angelegt. Bei dem metallischen Gegenstück kann es sich beispielsweise um ein Gehäuse der Vorrichtung oder eines Teils davon oder eine andere beliebige Elektrode oder einen anderen metallischen Gegenstand handeln, der analog wie der Draht der Elektrode 2 mit der Stimulatoreinheit 8 verbunden ist.

In einer weiteren Ausführungsform kann Elektrode 2 auch aus mehr als zwei einzelnen Drähten bestehen, die sowohl für die Ermittlung eines Messsignals im Gehirn als auch für die Stimulation herangezogen werden können. Beispielsweise können vier Drähte in einem Leiterkabel untergebracht sein, wobei zwischen verschiedenen Enden eine Potenzialdifferenz angelegt oder gemessen werden kann. Hierdurch lässt sich die Größe des abgeleiteten bzw. stimulierten Zielgebietes variieren.

Die Anzahl der Drähte, aus welchen sich die Elektrode aufbaut, ist nach oberen Werten hin lediglich durch die damit verbundene Dicke des in das Gehirn einzuführenden Kabels begrenzt, so dass möglichst wenig Hirnmaterial beschädigt werden soll. Handelsübliche Elektroden umfassen vier Drähte, es können jedoch auch fünf, sechs oder mehr Drähte, aber auch nur drei Drähte umfasst sein.

In einer bevorzugten Ausführungsform können mindestens zwei Elektroden 2 baulich vereint sein. Hierzu können beispielsweise n Drähte mit verschiedenen Kontaktstellen 14 zum Gehirn in einer Hirnelektrode vereinigt sein. Die Kontaktstellen 14 können im Wesentlichen übereinander und/oder radial zueinander verschoben angeordnet sein.

Die geeigneten Elektroden sind dem Fachmann bekannt und nicht auf die beispielhaft aufgeführten Elektroden beschränkt. Von dem Begriff Elektrode soll auch ein Chip umfasst sein, welcher Kontaktstellen 14 aufweist.

Für den Fall, dass die Elektrode 2 mehr als zwei Drähte umfasst, können mindestens zwei dieser Drähte auch als Sensor 3 fungieren, so dass in diesem Spezialfall eine Ausführungsform vorliegt, bei der die Elektrode 2 und der Sensor 3 in einem einzigen Bauteil vereint sind. Die Drähte der Elektrode 2 können unterschiedliche Längen haben, so dass sie in verschiedene Hirntiefen eindringen können. Besteht die Elektrode 2 aus n Drähten, so kann eine Stimulation über mindestens ein Paar von Drähten erfolgen, wobei bei der Paarbildung jede Unterkombination von Drähten möglich ist. Neben diesem Bauteil können zusätzlich nicht mit Elektrode 2 baulich vereinte Sensoren 3 vorhanden sein.

Die Einheit zur Signalverarbeitung und Steuerung 4 umfasst Mittel für eine univariate und/oder bivariate und/oder multivariate Datenverarbeitung, wie sie beispielsweise in "Detection of n:m Phase Locking from Noisy Data: Application to Magnetoencephalography" von P. Tass, et.al. in Physical Review Letters, 81,3291 (1998) beschrieben ist.

Vorzugsweise ist die Vorrichtung mit Mitteln ausgestattet, welche die Signale der Elektrode 2 und/oder der Sensoren 3 als pathologisch erkennen und im Falle des Vorliegens eines pathologischen Musters über die Elektrode 2 Reize abgeben, die bewirken dass die pathologische neuronale Aktivität entweder kurzfristig unterdrückt oder so modifiziert wird, dass sie der natürlichen, physiologischen Aktivität näher kommt. Die pathologische Aktivität unterscheidet sich von der gesunden Aktivität durch eine charakteristische Veränderung ihres Musters und/oder ihrer Amplitude, die dem Fachmann bekannt sind und die mit bekannten Methoden detektiert werden kann.

Die Mittel zum Erkennen des pathologischen Musters sind dabei ein Rechner, der die gemessenen Signale der Elektrode 2 und/oder des Sensors 3 verarbeitet und mit im Rechner gespeicherten Daten vergleicht. Der Rechner verfügt über einen Datenträger, welcher Daten speichert, die im Rahmen einer Eichprozedur ermittelt wurden. Beispielhaft können diese Daten ermittelt werden, indem in einer Serie von Testreizen die Stimulationsparameter systematisch variiert werden und der Erfolg der Stimulation über die Elektrode 2 und/oder den Sensor 3 mittels der Steuereinheit 4 ermittelt wird. Die Ermittlung kann durch uni- und/oder bi- und/oder multivariate Datenanalyse zur Kennzeichnung der Frequenzeigenschaften und der Interaktion (z. B. Kohärenz, Phasensynchronisation, Direktionalität und Reiz-Antwort-Beziehung) erfolgen, wie sie beispielsweise in P.A. Tass: "Phase resetting in Medicine and Biology. Stochastic Modelling and Data Analysis." Springer Verlag, Berlin 1999 offenbart ist.

Die erfindungsgemäße Vorrichtung umfasst daher vorzugsweise einen Rechner, welcher einen Datenträger beinhaltet, der die Daten des Krankheitsbildes trägt, mit den Messdaten vergleicht und im Falle des Auftretens pathologischer Aktivität ein Reizsignal an die Elektrode 2 abgibt, so dass eine Stimulation des Hirngewebes erfolgt. Die in dem Datenträger gespeicherten Daten des Krankheitsbildes können entweder personenspezifische, durch Eichung bestimmte optimale Stimulationsparameter sein oder ein Datenmuster, welches aus einem Patientenkollektiv bestimmt worden ist und typischerweise auftretende optimale Stimulationsparameter repräsentiert. Der Rechner erkennt das pathologische Muster und/oder die pathologische Amplitude.

Die Steuereinheit 4 kann beispielsweise einen Chip oder eine andere elektronische Vorrichtung mit vergleichbarer Rechenleistung umfassen.

Die Steuereinheit 4 steuert die Elektrode 2 vorzugsweise in folgender Weise an. Die Steuerdaten werden von der Steuereinheit 4 an einen optischen Sender für die Stimulation 5 weitergegeben, welcher über den Lichtleiter 6 den optischen Empfänger 7 ansteuert. Durch das optische Einkoppeln von Steuersignalen in den optischen Empfänger 7 wird eine galvanische Entkopplung der Stimulationssteuerung von der Elektrode 2 bewirkt. Dies bedeutet, dass eine Einstreuung von Störsignalen von der Einheit zur Signalverarbeitung und Steuerung 4 in die Elektrode 2 verhindert wird. Als optischer Empfänger 7 kommt beispielsweise eine Photozelle in Betracht. Der optische Empfänger 7 gibt die über den optischen Sender für die Stimulation 5 eingegebenen Signale an die Stimulatoreinheit 8 weiter. Über die Stimulatoreinheit 8 werden dann gezielte Stimuli über die Elektrode 2 an die Zielregion im Gehirn weitergegeben. Für den Fall, dass über die Elektrode 2 auch gemessen wird, wird ausgehend vom optischen Sender für die Stimulation 5 über den optischen Empfänger 7 auch ein Relais 9 angesteuert, was die Einstreuung von Störsignalen verhindert. Das Relais 9 oder der Transistor stellt sicher, dass die neuronale Aktivität unmittelbar nach jedem Stimulus wieder gemessen werden kann, ohne dass der Trennverstärker übersteuert. Die galvanische Entkopplung muss nicht zwingend durch eine optische Einkopplung der Steuersignale erfolgen, vielmehr können auch andere alternative Steuerungen verwendet werden. Diese können beispielsweise akustische Einkopplungen zum Beispiel im Ultraschallbereich sein. Eine störungsfreie Steuerung kann auch beispielsweise unter Zuhilfenahme geeigneter analoger oder digitaler Filter realisiert werden.

In einer weiteren Ausführungsform steht die erfindungsgemäße Vorrichtung vorzugsweise mit Mitteln zur Visualisierung und Verarbeitung der Signale sowie zur Datensicherung 13 über den Telemetrieempfänger 12 in Verbindung. Dabei kann die Einheit 13 über die oben erwähnten Verfahren zur uni- und/oder bi- und/oder multivariaten Datenanalyse verfügen.

In einer weiteren Ausführungsform kann die erfindungsgemäße Vorrichtung über den Telemetrieempfänger 13 mit einer zusätzlichen Referenzdatenbank in Verbindung stehen, um beispielsweise den Eichprozess zu beschleunigen.

Im Folgenden soll auf die erfindungsgemäße Funktionsweise der Vorrichtung sowie das erfindungsgemäße Verfahren eingegangen werden.

Die Beschreibung der Funktionsweise der Vorrichtung beschreibt implizit eine Vorrichtung und eine Steuerung sowie ein Verfahren zur Behandlung von Krankheiten, welche nach der angegebenen Funktionsweise arbeiten.

Erfindungsgemäß werden an die zerebralen Neuronen über mindestens zwei Kontaktstellen 14 niederfrequente Folgen von Hochfrequenzpulszügen, vorzugsweise kurzen Hochfrequenzpulszügen appliziert, welche aus Einzelpulsen bestehen.

Unter einer niederfrequenten Folge von Hochfrequenz-pulszügen im Sinne der Erfindung ist eine im Wesentlichen periodische Abfolge von Hochfrequenzpulszügen mit einer Frequenz von 0,1 Hz bis 50 Hz, vorzugsweise 1 Hz bis 10 Hz, besonders bevorzugt 2 Hz bis 8 Hz zu verstehen.

Im Folgenden sollen die möglichen Merkmale der Einzelpulse beschrieben werden:

Die Einzelpulse können eine Dauer von $\geq 0$ bis 10 $\mu$s, oder $\geq 0$ bis 50$\mu$s, $\geq 0$ bis 100$\mu$s, $\geq 0$ bis 200$\mu$s oder - weniger bevorzugt - bis zu 3000$\mu$s haben.

Unter einem elektrischen Einzelpuls wird ein dem Fachmann bekannter, im Wesentlichen ladungsneutraler einzelner elektrischer Puls verstanden.

Ladungsneutral im Sinne der Erfindung bedeutet, dass das Zeitintegral des Ladungseintrags im Wesentlichen null ist.

Die bevorzugte Amplitude der Einzelpulse liegt im Bereich von 0 bis 16 V, vorzugsweise zwischen 2 und 6 V.

Unter der Amplitude eines Einzelpulses wird das Maximum des Betrags des Zeitgangs des Ladungseintrags dieses Einzelpulses verstanden.

Im Folgenden werden die möglichen Merkmale eines Hochfrequenz-Pulszugs erläutert:

Unter einem kurzen Hochfrequenzpulszug im Sinne der Erfindung wird eine kurze hochfrequente Abfolge von elektrischen Einzelpulsen verstanden.

Die Dauer der Einzelpulse ist in einem Hochfrequenz-Pulszug lediglich beschränkt durch die zeitliche Distanz zum nachfolgenden Einzelpuls innerhalb desselben Hochfrequenz-Pulszugs.

Bei einer Frequenz des Hochfrequenz-Pulszugs von z. B. 130 Hz ergibt sich somit die Periode des Hochfrequenz-Pulszugs von ca. 7,69 ms als prinzipielle Obergrenze der Länge eines Einzelpulses.

Kurz bedeutet, dass diese hochfrequente Abfolge aus mindestens zwei, vorzugsweise 3,4,5,6,7,8,9,10,11,12, 13,14,15,16,17,18,19, 20, 50 oder bis zu 100 Einzelpulsen besteht.

Vorzugsweise liegt die Anzahl der Einzelpulse, aus denen ein kurzer Hochfrequenzpulszug besteht, im Bereich von 3 bis 15 Einzelpulsen.

Die prinzipielle Obergrenze der Anzahl der Einzelpulse in einem Hochfrequenz-Pulszug wird bestimmt durch die Periodendauer der periodischen niederfrequenten Abfolge, mit welcher die Hochfrequenz-Pulszüge appliziert werden.

Beträgt beispielsweise die Frequenz der niederfrequenten Abfolge von Hochfrequenz-Pulszügen 5 Hz, so kann die Dauer eines einzelnen Hochfrequenz-Pulszugs 200 ms nicht überschreiten. Bei einer Frequenz von 100 Hz des Hochfrequenz-Pulszugs und einer Dauer von 100 $\mu$s kann der einzelne Hochfrequenz-Pulszug prinzipiell maximal 20 Einzelpulse enthalten.

Da die Wirkung eines Hochfrequenz-Pulszugs von der Phase eines stimulierten neuronalen Rhythmus abhängt, sollte - um wirksam die Phasendynamik modulieren zu können - die Länge des Hochfrequenz-Pulszugs im wesentlichen maximal 3/4 so lang wie die Periode der niederfrequenten Abfolge sein und z.B. im Wesentlichen vorzugsweise 1/10 - 1/4 dieser Periode betragen.

Hochfrequent bedeutet im Sinne der Erfindung, dass die Frequenz vorzugsweise zwischen 50 bis 250 Hz, vorzugsweise zwischen 80 und 150 Hz besonders bevorzugt zwischen 100 und 140 liegt.

In einer weniger bevorzugten Ausführungsform können auch ladungsneutrale kurze Hochfrequenzpulszüge verwendet werden, die aus unterschiedlichen nicht ladungsneutralen Einzelpulsen bestehen.

Die Form der Einzelpulse, d. h. der zeitliche Verlauf des Ladungseintrages, kann dabei symmetrisch oder unsymmetrisch sein. Das heißt, bei diesen biphasischen Einzelpulsen können der kathodische und anodische Teil des Einzelpulses symmetrisch oder unsymmetrisch sein. Im symmetrischen Fall sind dabei der kathodische und der anodische Teil des Einzelpulses bis auf das Vorzeichen des Stromflusses identisch. Neben biphasischen Einzelpulsen können auch triphasische und multiphasische Einzelpulse appliziert werden.

Vorzugsweise ist die Form der Einzelpulse für alle Einzelpulse eines Hochfrequenz-Pulszugs gleich, sie kann jedoch auch für mindestens zwei Einzelpulse innerhalb eines Hochfrequenz-Pulszugs unterschiedlich sein.

Vorzugsweise ist die Amplitude für alle Einzelpulse gleich, sie kann jedoch auch für mindestens zwei Einzelpulse innerhalb eines Hochfrequenz-Pulszuges unterschiedlich sein.

Vorzugsweise ist die Dauer für alle Einzelpulse gleich, sie kann jedoch auch für mindestens zwei Einzelpulse innerhalb eines Hochfrequenz-Pulszuges unterschiedlich sein.

Die Amplitude der Hochfrequenzpulszüge kann in einer Größenordnung von 0 bis 16 V liegen. Vorzugsweise liegt die Amplitude der Hochfrequenzpulszüge zwischen 2 und 7 V. Der übliche Widerstand von Elektrode und Hirngewebe liegt beispielsweise im Bereich von 800 bis 1200 $\Omega$.

Die Frequenz eines Hochfrequenz-Pulszugs, der aus identischen Einzelpulsen besteht, ist die Rate, mit der die Einzelpulse appliziert werden. D. h. die Periode eines Hochfrequenz-Pulszugs ist der zeitliche Abstand zwischen dem Beginn aufeinander folgender Einzelpulse. Falls der Hochfrequenz-Pulszug mindestens zwei Einzelpulse enthält, die sich bezüglich ihrer Form und/oder Dauer unterscheiden, wird als Periode des Hochfrequenz-Pulszugs der zeitliche Abstand zwischen den Marker events aufeinander folgender Einzelpulse verstanden. Unter einem Marker event versteht man ein charakteristisches heranzuziehendes Merkmal, wie beispielsweise Beginn eines Einzelpulses, Ende eines Einzelpulses, bei bi- oder multiphasischen Einzelpulsen Beginn oder Ende einer bestimmten Phase eines Einzelpulses, Zeitpunkt der maximalen Amplitude, Zeitpunkt des Schwerpunkts des Betrags des Zeitgangs des Ladungseintrags, n-tes Maximum oder n-tes Minimum oder n-ter Wendepunkt des Zeitgangs des Ladungseintrags, wobei n eine positive ganze Zahl ist.

Innerhalb eines Hochfrequenz-Pulszugs muss die Periode nicht konstant sein. Vielmehr können mindestens zwei der Perioden innerhalb eines Hochfrequenz-Pulszugs verschieden sein. D.h. die momentane, auf die einzelne Periode bezogene Frequenz eines Hochfrequenz-Pulszugs kann innerhalb eines Hochfrequenz-Pulszugs variieren. Die Reihenfolge der einzelnen Perioden in einem Hochfrequenz-Pulszug kann durch deterministische Gesetzmäßigkeiten und/oder stochastische Gesetzmäßigkeiten und/oder Kombinationen von beiden gegeben sein.

Ein Hochfrequenzpulszug kann auch aus Einzelpulsen bestehen, von denen mindestens zwei Einzelpulse unterschiedliche Form und/oder unterschiedliche Amplitude und/oder unterschiedliche Dauer haben. Die Form und/oder die Dauer und/oder die Amplitude der Einzelpulse können durch deterministische und/oder stochastische Gesetzmäßigkeiten und/oder Kombinationen von beiden gegeben sein. Bei einer Kombination von stochastischen und deterministischen Gesetzmäßigkeiten handelt es sich um einen funktionellen Zusammenhang, bei dem deterministische und stochastische Terme funktionell, z. B. durch Addition oder Multiplikation, miteinander verbunden sind. Zum Beispiel kann die Amplitude des j-ten Einzelpulses durch f(j) gegeben sein, wobei f eine deterministische Funktion und/oder ein stochastischer Prozess und/oder eine Kombination von beiden ist.

Im Folgenden sollen Möglichkeiten der über einen einzelnen Stimulationskontakt applizierten Hochfrequenz-Pulszüge innerhalb der niederfrequenten Abfolge von Hochfrequenz-Pulszügen beschrieben werden:

Eine niederfrequente Abfolge von kurzen Hochfrequenz-pulszügen umfasst vorzugsweise 2-100, besonders bevorzugt 2-20 oder 2-10 Hochfrequenzpulszüge.

Die niederfrequente Abfolge von kurzen Hochfrequenz-pulszügen besteht vorzugsweise aus einer periodischen Abfolge von kurzen Hochfrequenzpulszügen, deren Frequenz im Wesentlichen der pathologischen Frequenz - beispielsweise beim Morbus Parkinson ca. 5 Hz - oder einem kleinen ganzzahligen Vielfachen, z. B. dem doppelten, dem dreifachen oder dem vierfachen, entspricht. Die Periode der niederfrequenten Abfolge von kurzen Hochfrequenz-Pulszügen wird im Folgenden als Stimulationsperiode bezeichnet. Unter niederfrequent im Sinne der Erfindung sind Frequenzen von 0,5-50 Hz, bevorzugt 2-20 Hz, besonders bevorzugt 2-10 Hz zu verstehen.

Eine niederfrequente Abfolge von kurzen Hochfrequenz-pulszügen besteht vorzugsweise aus denselben Hochfrequenzpulszügen. Mindestens 2 Hochfrequenzpulszüge einer solchen niederfrequenten Abfolge können sich aber auch bezüglich ihres Musters unterscheiden.

Das Muster eines Hochfrequenzpulszugs umfasst folgende Eigenschaften:

    A) die Anzahl der Einzelpulse,
    B) die Form der Einzelpulse,
    C) die Dauern der einzelnen Einzelpulse,
    D) die Amplituden der einzelnen Einzelpulse,.
    E) die Perioden zwischen den einzelnen Einzelpulsen.

Innerhalb einer niederfrequenten Abfolge von kurzen Hochfrequenz-Pulszügen kann das Muster von Hochfrequenzpulszug zu Hochfrequenz-Pulszug deterministisch und/oder stochastisch und/oder kombiniert deterministisch-stochastisch variiert werden. Insbesondere kann innerhalb einer niederfrequenten Abfolge von kurzen Hochfrequenzpulszügen die Frequenz von mindestens zwei kurzen Hochfrequenzpulszügen verschieden sein. Vorzugsweise haben alle Hochfrequenzpulszüge einer niederfrequenten Folge von Hochfrequenz-Pulszügen die gleiche Anzahl von Einzelpulsen. Es können aber auch mindestens zwei Hochfrequenzpulszüge aus einer unterschiedlichen Anzahl von Einzelpulsen bestehen.

Vorzugsweise haben alle Hochfrequenz-Pulszüge einer niederfrequenten Folge von Hochfrequenz-Pulszügen die gleiche Frequenz. Es können aber auch mindestens zwei Hochfrequenzpulszüge aus Einzelpulsen unterschiedlicher Frequenz bestehen.

Vorzugsweise ist die Amplitude der Einzelpulse für alle Hochfrequenzpulszüge einer niederfrequenten Folge von Hochfrequenz-Pulszügen gleich, sie kann jedoch auch für mindestens zwei Hochfrequenz-Pulszüge unterschiedlich sein.

Zeitliche Struktur der niederfrequenten Abfolge von Hochfrequenz-Pulszügen:

Die niederfrequente Abfolge von Hochfrequenz-Pulszügen muss nicht streng periodisch sein. Innerhalb einer niederfrequenten Abfolge von Hochfrequenz-Pulszügen kann der zeitliche Abstand aufeinander folgender Hochfrequenz-Pulszüge deterministisch und/oder stochastisch und/oder deterministisch-stochastisch variiert werden.

Innerhalb einer niederfrequenten Abfolge von HF-Pulszügen können der zeitliche Abstand aufeinander folgender Hochfrequenz-Pulszüge und/oder das Muster des jeweiligen Hochfrequenz-Pulszugs deterministisch und/oder stochastisch und/oder kombiniert deterministisch-stochastisch variiert werden.

Die Steuerung der erfindungsgemäßen Vorrichtung kann die niederfrequente Abfolge von HF-Pulszügen dabei in folgender Weise regeln:

Mit $H_j$ wird das Muster des j-ten Hochfrequenz-Pulszugs bezeichnet.

Mit pj wird die Pause nach dem j-ten Hochfrequenz-Pulszug bezeichnet.

Eine z.B. aus 4 Hochfrequenz-Pulszügen bestehende niederfrequente Abfolge von Hochfrequenz-Pulszügen hat dementsprechend folgende Struktur:

$$H_1\ p_1\ H_2\ p_2\ H_3\ p_3\ H_4\ p_4$$

Die Steuerung der erfindungsgemäßen Vorrichtung kann die Muster $H_j$ (j=1,2,..) und die Pausen $p_j$ (j=1,2..) gemäß eines deterministischen und/oder stochastischen und/oder kombiniert deterministisch-stochastischen Algorithmus bestimmen.

$H_j$ ist dabei folgender Vektor:

$$H_j\ =\ (m_j,\ \underline{P}_{jk}\ ,\ \underline{Z}_{j1},\ \underline{Z}_{j2},\ldots\underline{Z}_{jm}),$$

Mit Unterstrich (-) als Symbol für einen Vektor,
$m_j$ = Anzahl der Einzelpulse im j-ten Hochfrequenz-Pulszug,
$\underline{P}_{jk}$ = Dauer der Pause nach dem k-ten Einzelpuls im j-ten Hochfrequenz-Pulszug,
$\underline{Z}_{jk}$ = Vektor der die Eigenschaften $\underline{Z}_{jk}$ =($\underline{Z}_{jk}^{(1)}$, $\underline{Z}_{jk}^{(2)}$,.... $\underline{Z}_{jk}^{(m)}$) des k-ten Einzelpulses im j-tem Hochfrequenz-Pulszug bestimmt.

Im allgemeinsten Fall ist $Z_{jk}$ der Zeitgang des k-ten Einzelpulses im j-ten Hochfrequenz-Pulszug. Die Länge des Vektors wird dabei von der Abtastrate bestimmt.

Vorzugsweise wird das Inventar möglicher Formen der Einzelpulse eingeschränkt, so dass die Form mit deutlich weniger Parametern eindeutig gekennzeichnet werden kann, was zu einer einfacheren Steuerung führt.

Werden z.B. nur biphasische, rechteckförmige Einzelpulse verwendet, so besteht $\underline{Z}_{jk}$ aus nur sechs Komponenten:

1. Beginn des Einzelpulses, z. B. $\underline{Z}_{jk}^{(1)}$ = 0$\mu$s
2. Ende des Einzelpulses, z. B. $\underline{Z}_{jk}^{(2)}$= 500$\mu$s
3. Binäre Informationen kodieren, ob die erste Phase des Einzelpulses kathodisch, die zweite anodisch ($\underline{Z}_{jk}^{(3)}$= 1) oder die erste Phase des Einzelpulses anodisch und die zweite Phase kathodisch ($\underline{Z}_{jk}^{(3)}$= 0) ist,
4. Dauer des ersten Phase (z. B. $\underline{Z}_{jk}^{(4)}$= 100$\mu$s)
5. Amplitude der ersten Phase (z. B. $\underline{Z}_{jk}^{(5)}$= 4V),
6. Amplitude der zweiten Phase (z. B. $\underline{Z}_{jk}^{(6)}$= 1V).

Erfindungsgemäß werden die niederfrequenten Abfolgen von kurzen Hochfrequenz-Pulszügen über mindestens zwei Kontaktstellen 14 appliziert, welche sich auf mindestens einer Elektrode 2 befinden.

Gemäß dem Stand der Technik, wie er in der Schrift WO 2004/093981 A1 beschrieben ist, kann die Applikation der kurzen Hochfrequenz-Pulszüge über n Elektroden bzw. Kontaktstellen 14 mit einer zeitlichen Verschiebung der einzelnen Hochfrequenz-Pulszüge von im wesentlichen T/n erfolgen, wobei T die Periode der niederfrequenten Abfolge der Hochfrequenz-Pulszüge ist. Die Abweichung von T/n kann dabei $\pm$ 0,25T und mehr, bis zu $\pm$0,75T betragen, ohne dass die erfindungsgemäße Wirkung verloren geht.

Beispielsweise werden mit 2,3,4,5 oder 6 Elektroden 2 bzw. Kontaktstellen 14 die jeweiligen Hochfrequenz-Pulszüge mit einer zeitlichen Verschiebung von T/2, T/3, T/4, T/5, oder T/6 appliziert.

Beispielsweise wird im Fall von drei Elektroden 2 mit jeweils einer Kontaktstelle 14 bzw. Kontaktstellen 14 zuerst zurzeit t=0s ein Hochfrequenz-Pulszug über Elektrode Nummer eins bzw. Kontaktstelle 14 (Nr.1) appliziert, zur Zeit t = T/3 wird ein Hochfrequnz-Pulszug über Elektrode Nummer zwei bzw. Kontaktstelle 14 (Nr.2) verabreicht und zur Zeit t = 2T/3 schließlich ein Hochfrequenz-Pulszug über Elektrode Nummer drei bzw. Kontaktstelle 14 (Nr.3) appliziert. Zur Ansteuerung der einzelnen Elektroden beziehungsweise der Kontaktstellen 14 kann aber auch jede andere mögliche Reihenfolge verwendet werden. D. h. statt der beispielhaft gewählten Reihenfolge 1-2-3 wären auch 1-3-2, 2-1-3, 2-3-1, 3-1-2 und 3-2-1 mögliche Reihenfolgen.

Wird bei n Hochfrequenz-Pulszügen, die sequenziell appliziert werden, ein Paar von Hochfrequenz-Pulszügen mit einer zeitlichen Verschiebung von ca. einer halben Periode appliziert, so kann alternativ folgendes gemacht werden:

1. Der zuerst applizierte Hochfrequenz-Pulszug wird unverändert appliziert. Der um eine im Wesentlichen halbe Periode verzögerte Hochfrequenz-Pulszug wird ohne Zeitverzögerung aber mit Umkehr der Polarität appliziert.

2. Der spätere Hochfrequenz-Pulszug wird unverändert appliziert. Der frühere Hochfrequenz-Pulszug wird mit einer zeitlichen Verzögerung von ca. einer im Wesentlichen halber Periode aber unter Polaritätsumkehr appliziert.

Erfindungsgemäß wird unter der Zielpopulation die unmittelbar durch eine implantierte Elektrode stimulierte Nervenzellpopulation verstanden.

Eine Zielpopulation wird durch eine in ihr oder nahe bei ihr implantierte Elektrode direkt stimuliert.

Die Nervenzellpopulation, welche krankhaft synchron aktiv ist, wird als zu desynchronisierendes Areal oder als zu desynchronisierende Nervenzellpopulation oder als zu desynchronisierende Neuronenpopulation bezeichnet. Letztere ist nicht an anatomische Grenzen gebunden. Vielmehr kann darunter auch mindestens eine Komponente, bestehend aus der Gruppe

- mindestens ein Teil von mindestens einem anatomischen Areal,
- mindestens ein vollständiges anatomisches Areal, verstanden werden.

Das zu desynchronisierende Areal kann entweder direkt oder indirekt stimuliert werden.

Direkte Stimulation über eine Stimulationselektrode 2: In diesem Fall befindet sich die Stimulationselektrode 2 in dem zu desynchronisierenden Areal. Diese Elektrode 2 beeinflusst dabei die Zielpopulation, welche sich in dem zu desynchronisierenden Areal befindet.

Indirekte Stimulation über eine Stimulationselektrode 2:

In diesem Fall wird das zu desynchronisierende Areal mittels Elektrode 2 nicht direkt stimuliert. Vielmehr wird über die Stimulationselektrode 2 eine Zielpopulation oder ein Faserbündel, welche mit dem zu desynchronisierenden Areal funktionell eng verbunden sind, stimuliert. Hierbei wird der Stimulationseffekt auf das zu desynchronisierende Areal vorzugsweise über anatomische Verbindungen fortgeleitet. Für die indirekte Stimulation soll als Oberbegriff für Zielpopulation und Faserbündel der Begriff Zielareal eingeführt werden. Von dem Begriff Zielareal sollen im Folgenden die mit dem zu desynchronisierenden Areal funktionell eng verbundene Neuronenpopulation und das verbindende Faserbündel verstanden werden.

Die Dauer zwischen Beginn der Stimulusapplikation über die j-te Elektrode 2 bzw. Kontaktstelle 14 und dem ersten Maximum der Reizantwort bzw. des Betrags der Reiz-antwort, $\tau j^{(k)}$, wird für jede einzelne Reizapplikation bestimmt. Bei $\tau j^{(k)}$ steht der Index j für die j-te Elektrode 2 bzw. Kontaktstelle 14, während der Index k für den k-ten applizierten Stimulus steht. Hieraus wird dann für jede Stimulationselektrode 2 bzw. Kontaktstelle 14, über die indirekt stimuliert wird, separat die mittlere Dauer zwischen Reizbeginn und Reizantwort nach folgender Formel 1 bestimmt:

$$\bar{\tau}_j = \frac{1}{L_j} \sum_{k=1}^{L_j} \tau_j^{(k)} \ .$$

Formel 1

Hierbei ist $L_j$ die Anzahl der über die j-te Stimulationselektrode 2 bzw. Kontaktstelle 14 applizierten Reize. $L_j$ kann, aber muss nicht für alle Stimulationselektroden 2 bzw. Kontaktstellen 14, über die indirekt stimuliert wird, gleich sein.

Für die desynchronisierende Stimulation wird die auf diese Weise bestimmte Leitungszeit $\bar{\tau}_j$ in folgender Weise berücksichtigt:

Würde bei direkter Stimulation der zu desynchronisierenden Neuronenpopulation zur Zeit t über die j-te Stimulationselektrode 2 bzw. Kontaktstelle 14 ein Reiz appliziert, so wird bei indirekter Stimulation, das heißt, bei einer Stimulation die nicht direkt in der zu desynchronisierenden Neuronenpopulation, sondern in einem damit verbundenen Faserbündel und/oder einer damit verbundenen Neuronenpopulation erfolgt, über die j-te Stimulationselektrode 2 bzw. Kontaktstelle 14 der Reiz zur Zeit $t - \bar{\tau}_j$ verabreicht.

In einer besonders bevorzugten Ausführungsform wird innerhalb einer über mehrere Elektroden 2 bzw. Kontakt stellen 14 verabreichten niederfrequenten Abfolge von Hochfrequenz-Pulszügen die Reihenfolge der Reizapplikation über die verschiedenen Elektroden/ Kontaktstellen 14 deterministisch und/oder stochastisch und/oder kombiniert deterministisch-stochastisch variiert.

Die Struktur einer niederfrequenten Abfolge von Sequenzen:

Die Abfolge der über n Elektroden 2 bzw. Kontaktstellen 14 zeitverzögert applizierten einzelnen Hochfrequenz-Pulszüge wird als Sequenz von Hochfrequenz-Pulszügen bezeichnet.

Zeitliche Ansteuerung der Sequenzen:

Die erfindungsgemäße Vorrichtung kann die Zeitpunkte aufeinander folgender Sequenzen so steuern, dass diese streng periodisch appliziert werden. In diesem Fall handelt es sich um eine niederfrequente Abfolge von Sequenzen. Weisen dabei mindestens zwei dieser Sequenzen eine unterschiedliche Reihenfolge der Elektrodenansteuerung an, so können nicht alle über die n Elektroden 2 bzw. Kontaktstellen 14 verabreichten niederfrequenten Abfolgen von Hochfrequenz-Pulszügen dieselbe Periode wie die niederfrequente Abfolge der Sequenzen aufweisen.
Die zeitliche Abstände zwischen aufeinander folgenden Sequenzen können deterministisch und/oder stochastisch und/oder kombiniert deterministisch-stochastisch gewählt werden.

Auswahl der m Elektroden von insgesamt n Elektroden bzw. Kontaktstellen, über die in einer bestimmten Sequenz stimuliert wird; m = n:

Erfindungsgemäß werden bei einer Sequenz nur $m \geq 2$ der insgesamt n ($m \leq n$) Elektroden 2 bzw. Kontaktstellen 14 mit einer Zeitverzögerung von T/m angesteuert.
Als Konfiguration einer Sequenz wird (i) die Auswahl der m = 2 Elektroden 2 bzw. Kontaktstellen 14 von insgesamt n ($m \leq n$) Elektroden bzw. Kontaktstellen 14 und (ii) deren Reihenfolge bei der Ansteuerung verstanden. Zum Beispiel kann die Konfiguration einer Sequenz bei insgesamt n=4 Elektroden 2 bzw. Kontaktstellen 14 2-3-1 sein d.h. es werden nur die Elektroden 2 bzw. Kontaktstellen 14 1,2 und 3 angesteuert und zwar in der Reihenfolge 2-3-1.
Die Konfiguration kann innerhalb einer Reihenfolge von Sequenzen deterministisch und/oder stochastisch und/ oder kombiniert deterministisch-stochastisch variiert werden.
Insgesamt können innerhalb einer Reihenfolge von Sequenzen deren Konfigurationen, die zeitlichen Abstände zwischen aufeinander folgenden Sequenzen und die Muster der in den jeweiligen Sequenzen verwendeten Hochfrequenz-Pulszüge aufeinander abgestimmt deterministisch und/oder stochastisch und/oder kombiniert deterministisch-stochastisch variiert werden.
Die Struktur der applizierten Sequenzen ist somit von der Form

$$S_1 \, P_1 \, S_2 \, P_2 \, S_3 \, P_3 \, .... \, S_k \, P_k.$$

Sj bezeichnet die Anzahl und die Art inklusive Konfiguration und Muster der verwendeten HF-Pulszüge der in der j-ten Reihenfolge von Sequenzen verwendeten Sequenzen. $P_j$ bezeichnet die Dauer der nachfolgenden Pause.
In einer möglichen, aber nicht bevorzugten Ausführungsform der erfindungsgemäßen Vorrichtung wird die Abfolge

$$S_1 \, P_1 \, S_2 \, P_2 \, S_3 \, P_3 \, .... \, S_k \, P_k$$

ohne Bedarfssteuerung, d. h. ohne Rückmeldung stochastisch und/oder deterministisch und oder kombiniert deterministisch-stochastisch bestimmt.
Im allereinfachsten Fall handelt es sich bei $S_1 \, P_1 \, S_2 \, P_2$ ...... um eine streng periodische Abfolge $S_1 \, P_1 \, S_1 \, P_1$....
In einer bevorzugten Ausführungsform weist die erfindungsgemäße Vorrichtung eine Bedarfssteuerung auf. Hierzu wird über mindestens eine der Stimulationselektroden und/oder mindestens eine zusätzliche Elektrode 2 bzw. Kontaktstelle 14 (Sensor 3) die neuronale Aktivität gemessen, mittels univariaten und/oder bivariaten und/oder multivariaten Datenanalyseverfahren verarbeitet.
In Abhängigkeit der damit erzielten Ergebnisse wird die Abfolge $S_1 \, P_1 \, S_2 \, P_2$... in einer Weise modifiziert, dass mit möglichst wenig Stimulationsstrom eine möglichst effiziente Desynchronisation erzielt wird.
Hierbei gibt es mehrere Möglichkeiten:

(i) Bedarfsgesteuertes Timing:

Die Sequenz-Reihenfolgen $S_1$, $S_2$, $S_3$, etc. werden nicht geregelt, d. h. in Abhängigkeit vom Feedback-Signal und/oder von den Feedback-Signalen modifiziert. Es wird vorzugsweise ausschließlich die Dauer der Pausen $P_1$, $P_2$, $P_3$,...etc. bedarfsgesteuert geregelt, d. h. wann immer ein Schwellenwert des pathologischen Merkmals detektiert wird, wird die Pause $P_k$ beendet und die darauf folgende Sequenz-Reihenfolge $S_{k+1}$ appliziert.

Im einfachsten Fall wird immer dieselbe Sequenz-Reihenfolge $S_1$ bedarfsgesteuert mit unterschiedlichen Pausen appliziert:

$$S_1\ P_1\ S_1\ P_2\ S_1\ P_3\ S_1\ P_4\ .......$$

(ii) Bedarfsgesteuerte Variation der Sequenz-Reihenfolge $S_1$, $S_2$, $S_3$ etc. ohne Regelung der Pausen $P_1$, $P_2$, $P_3$ etc.:

Während der Pausen $P_1$, $P_2$, $P_3$ etc. wird die Ausprägung des pathologischen Merkmals gemessen. In Abhängigkeit hiervon wird die jeweils nachfolgende Sequenz-Reihenfolge $S_k$ bzw. werden die jeweils nachfolgenden Sequenz-Reihenfolgen $S_K$, $S_{k+1}$, $S_{k+2}$, ....., $S_{k+q}$ geregelt, wobei q eine positive ganze Zahl ist.

In einer einfachen Ausführungsform wird während jeweils konstanten Pausen die Ausprägung des pathologischen Merkmals gemessen und in Abhängigkeit hiervon die Amplitude der Hochfrequenz-Pulszüge der darauf folgenden Sequenz-Reihenfolge $S_k$ geregelt.

Sei $X_{k-1}$ die Variable, welche die Ausprägung des pathologischen Merkmals während der Pause $P_{k-1}$ beschreibt, so kann in einer einfachen Ausführungsform beispielsweise die Amplitude $A_k$ aller Hochfrequenz-Pulszüge der Sequenz-Reihenfolge $S_k$ in Abhängigkeit von $X_{k-1}$ geregelt werden.

In dieser Anmeldung wird unter dem Begriff "$A_k$ wird in Abhängigkeit von $X_{k-1}$ geregelt" folgendes verstanden.

$A_k$ wird mit Methoden der Regelungstechnik, die dem Fachmann bekannt sind, in Abhängigkeit von $X_{k-1}$ geregelt. Z.B. wird $A_k$ bestimmt gemäß $A_k=F(X_{k-1})$, wobei F eine Funktion ist, welche stückweise linear oder nichtlinear ist und $A_k$ in jedem Fall nach oben d.h. zu maximal Werten von z.B. 5V begrenzt ist um eine zu starke Stimulation zu verhindern.

Bei dieser beispielhaften Ausführungsform ergibt sich die Abfolge

$$S_1\ P_1\ S_2\ P_1\ S_3\ P_1\ S_4\ P_1.......\ ,$$

wobei zwischen die Sequenz-Reihenfolgen $S_1$, $S_2$, $S_3$ ...... etc. jeweils die gleiche Pause $P_1$ eingeschoben wird. Die Sequenz-Reihenfolgen $S_1$, $S_2$, $S_3$ ...... unterscheiden sich nur bezüglich der Amplitude $A_k$ ihrer Hochfrequenz-Pulszüge.

In einer weiteren, alternativen einfachen Ausführungsform wird die Bedarfssteuerung der Sequenz-Reihenfolgen $S_1$, $S_2$, $S_3$ etc. so durchgeführt, dass nicht die Amplitude der Hochfrequenz-Pulszüge der Sequenz-Reihenfolge $S_1$, $S_2$, $S_3$ etc. sondern die Länge der Sequenz-Reihenfolgen $S_1$, $S_2$, $S_3$ etc. in Anhängigkeit von der Ausprägung des pathologischen Merkmals $X_k$ geregelt wird. Z.B. kann es in einer einfachen Ausführungsform eine "Mutter-Sequenz-Reihenfolge" $S_M$ geben, welche aus einer Abfolge von Sequenzen $Seq_1$, $Seq_2$ etc. mit dazwischen geschobenen Pausen $Pa_1$, $Pa_2$ etc. besteht und zwar von der Form

$$S_M = Seq_1,\ Pa_1,\ Seq_2,\ Pa_2,\ Seq_3,\ Pa_3\ ...etc.$$

In Anhängigkeit von der Ausprägung des pathologischen Merkmals $X_k$ wird die Länge L von $S_M$ geregelt, wobei $S_M$ der Länge L bedeutet:

$$S_M = Seq_1,\ Pa_1,\ Seq_2,\ Pa_2,\ Seq_3,\ Pa_3,\ Seq_L.$$

Im einfachsten Fall sind die Sequenzen $Seq_1$, $Seq_2$, etc. identisch, so dass $S_M$ der Länge L eine zeitlich äquidistante Abfolge von L Einzelsequenzen $Seq_1$ ist.

(iii) Kombinierte Regelung, d. h. bedarfsgesteuerte Variation der Pausen $P_1$, $P_2$, etc. und bedarfsgesteuerte Variation der Sequenz-Reihenfolge S1, $S_2$ etc.:

In einer einfachen Ausführungsform der erfindungsgemäßen Vorrichtung wird z.B. die Länge $1_k$ der Hochfrequenz-Pulszüge in der Sequenz-Reihenfolge $S_k$ in Abhängigkeit von der Ausprägung des von der Ausprägung des pathologischen Merkmals $X_{k-1}$ während der Pause $P_{k-1}$ geregelt.

Um die Sequenz-Reihenfolgen $S_1$, $S_2$ etc. in einer beispielhaften Ausführungsform streng periodischen offsets (d. h. Endpunktes der Stimulation) applizieren zu können - was für eine rasche Desynchronisation vorteilhaft ist - müssen die Längen der Pausen $P_1$, $P_2$, etc. entsprechend kompensatorisch variiert werden. Lange Hochfrequenz-Pulszüge in

$S_k$ führen zu einer Verlängerung der Sequenz-Reihenfolge $S_k$. Damit der Offset von $S_k$ das Ende des letzten Hochfrequenz-Pulszugs von $S_k$, gemäß einer strengen Periodizität erfolgen kann, muss die voran stehende Pause $P_{k-1}$ entsprechend verkürzt werden. Ergibt sich also folgende Abfolge: $S_1$, $P_1$, $S_2$, $P_2$, $S_3$, $P_3$, ..., wobei die $S_1$, $S_2$ etc. sich lediglich bezüglich der Länge ihrer Hochfrequenz-Pulszüge unterscheiden und $P_{k-1}$ jeweils so gewählt wird, dass der Offset (d. h. der Zeitpunkt des Endes) - oder weniger bevorzugt - der Onset (d. h. der Zeitpunkt des Beginns) von $S_k$ streng periodisch erfolgt.

In einer weiteren Ausführungsform der erfindungsgemäßen Vorrichtung kann neben der Länge der Hochfrequenz-Pulszüge zusätzlich noch die Länge der Pausen $P_1$, $P_2$ etc. variiert werden.

In einer beispielhaften Ausführungsform der erfindungsgemäßen Vorrichtung wird die oben ausgeführte Regelung der Länge $l_k$ der Hochfrequenz-Pulszüge der Sequenz-Reihenfolge $S_k$ dadurch ergänzt, dass bei Unterschreiten der Ausprägung $X_{k-1}$ unter einen bestimmten Schwellenwert $X_{min}$ des pathologischen Merkmals während der Pause $P_{k-1}$ die Pause $P_{k-1}$ verlängert wird.

Die Verlängerung der Phase $P_{k-1}$ kann beispielsweise gequantelt erfolgen. D.h. $P_{k-1}$ wird um eine Periode T verlängert, welche typischerweise der Periode der pathologischen neuronalen Aktivität oder einem kleinen ganzzahligen Vielfachen davon entspricht. Unterschreitet nach Verlängerung von $P_{k-1}$ die Ausprägung $X_{k-1}$ weiterhin $X_{min}$, so wird $P_{k-1}$ nochmals um T verlängert u.s.w., bis schließlich $X_{k-1}$ $X_{min}$ überschreitet. Dann wird die Länge der Hochfrequenz-Pulszüge in $S_k$ in Abhängigkeit von $X_{k-1}$ wie oben beschrieben geregelt.

Für die oben beschriebene Bedarfssteuerung verfügt die erfindungsgemäße Vorrichtung vorzugsweise über Mittel, welche die Ausprägung des pathologischen Merkmals bestimmen. Hierzu wird über Sensor 3 das Feedback-Signal gemessen, welches die Aktivität der zu desynchronisierenden Neuronenpopulation darstellt. Dieses Feedback-Signal wird an die Einheit 4 zur Signalverarbeitung und/oder Regelung weitergeleitet, die als Mittel zum Erkennen eines pathologischen Merkmals fungiert. Sobald die Einheit 4 zur Signalverarbeitung und/oder Regelung im Feedback-Signal ein pathologisches Merkmal erkennt, wird ein Reiz appliziert. Unter einem pathologischen Merkmal sind beispielsweise folgende Eigenschaften des Feedback-Signals zu verstehen:

a) Die Amplitude des Feedback-Signals überschreitet einen Schwellenwert. Die erfindungsgemäße Vorrichtung ist daher in einer bevorzugten Ausführungsform mit Mitteln zum Erkennen eines Schwellenwertes des Feedback-Signals ausgestattet. In diesem Fall wird vorzugsweise das Feedback-Signal selbst oder sein Betrag oder seine Amplitude mit dem Schwellenwert verglichen. Das Mittel zum Erkennen des Schwellenwertes kann bei dieser Ausführungsform so programmiert sein, dass es beispielsweise das Feedback-Signal selbst und/oder sein Betrag und/oder seine Amplitude mit dem Schwellenwert vergleicht. Die Bestimmung der Amplitude erfolgt entweder in einer einfachen Version mittels Bestimmung des Betrags des Signals, oder mit Bandpassfilterung und nachfolgender HilbertTransformation oder Wavelet-Analyse. Die Einheit 4 zur Signalverarbeitung und/oder Regelung ist in diesem Fall so programmiert, dass sie eine Bestimmung des Betrags des Signals und/oder eine Bandpassfilterung mit Hilberttransformation und/oder eine Wavelet-Analyse durchführen kann. Das Feedback-Signal oder sein Betrag wird besonders bevorzugt verwendet, da die Berechnung der Amplitude einen deutlich höheren Rechenaufwand bedeutet, und die Genauigkeit dieser Berechnung von der richtigen Auswahl algorithmischer Parameter abhängt. Außerdem kann die Bestimmung der Amplitude nicht auf einem einzelnen Messwert des Feedback-Signals durchgeführt werden, sondern muss in einem hinreichend großen, dem Fachmann bekannten Zeitintervall durchgeführt werden. Durch diese Form der Analyse des Feedback-Signals in einem gleitenden Zeitfenster ist die Erkennung des pathologischen Merkmals etwas verzögert.

Die unter a) beschriebene Form der Analyse der Form des Feedback-Signals ist anzuwenden, wenn über Sensor 3 ausschließlich bzw. überwiegend die zu desynchronisierende pathologische Aktivität gemessen wird.

b) Falls über Sensor 3 neben dieser Aktivität zusätzlich noch nicht krankheitsspezifische Aktivität, zum Beispiel aus anderen Neuronenpopulationen, gemessen wird, muss bei der Analyse des Feedback-Signals ein weiterer algorithmischer Schritt eingefügt werden. Da die krankheitsspezifische Aktivität typischerweise in einem Frequenzbereich auftritt, der von dem Frequenzbereich der nicht krankheitsspezifischen Aktivität verschieden ist, genügt es hierzu vorzugsweise eine Abschätzung der Aktivität im krankheitsspezifischen Frequenzbereich durchzuführen. Die Frequenz der krankheitsspezifischen Aktivität wird beispielsweise durch eine Bestimmung der zeitlichen Differenz von aufeinander folgenden Triggerpunkten durchgeführt. Triggerpunkte sind charakteristische Punkte wie Maxima, Minima, Wendepunkte und Nulldurchgänge. Vorzugsweise wird diese Analyse in einem gleitenden Zeitfenster durchgeführt, wobei der Mittelwert von mehreren zeitlichen Differenzen gebildet wird, wodurch die Stabilität der Frequenzschätzung erhöht wird. Alternativ kann die Frequenzschätzung auch mit den dem Fachmann bekannten spektralen Schätzmethoden und anderen Frequenzschätzern bestimmt werden. Hierzu verfügt die erfindungsgemäße Vorrichtung in einer besonderen Ausführungsform Mittel zur Abschätzung der Aktivität im krankheitsspezifischen Frequenzbereich, wie spektrale Schätzmethoden, Wavelet-Analyse u.s.w. Dies wird beispielsweise durch eine Frequenzanalyse durch Mittel zum Durchführen einer Frequenzanalyse realisiert. Es kann beispielsweise die spektrale Energie im krankheitsspezifischen Frequenzbereich in einem gleitenden Fenster bestimmt werden. Alternativ kann nach Bandpassfilterung die Amplitude im krankheitsspezifischen Frequenzbereich durch Bestimmung des Maximums des bandpassgefilterten Signals oder durch Bestimmung des Mittelwerts des Betrags des bandpassgefilterten Signals oder mit nachfolgender HilbertTransformation oder mittels Wavelet-Analyse ermittelt werden. Hierzu umfasst die erfindungsgemäße Vorrichtung beispielsweise Mittel zur Bandpassfilterung der Amplitude und Mittel zur Bestimmung des Maximums des bandpassgefilterten Signals und/oder Mittel zur Bestimmung des Mittelwerts des Betrags des bandpassgefilterten Signals und/oder Mittel zur Durchführung einer Hilberttransformation und/oder einer Wavelet-Analyse.

Die erfindungsgemäße Vorrichtung kann mit einem Schwellwert-Kriterium wie oben beschrieben arbeiten. Zum Beispiel kann in Abhängigkeit von der Ausprägung eines pathologischen Merkmals die Stimulation modifiziert werden. Insbesondere kann die Stimulation so lange sistieren, bis das pathologische Merkmal einen Schwellenwert überschreitet. Das Überschreiten des Schwellenwertes signalisiert in dieser Ausführungsform den Bedarf für eine erneute Stimulation.

Feststellung des Bedarfs:

Aus mindestens zwei Gründen gibt es keine eineindeutige Beziehung zwischen der Ausprägung des pathologischen Merkmals und der Ausprägung der krankheitsspezifischen Symptome. Zum einen bedingt die Entfernung von Sensor 3 zu dem Areal, in welchem das Feedback-Signal generiert wird, die Amplitude im krankheitsspezifischen Frequenzbereich. Zum anderen ist eine bestimmte Ausprägung des krankheitsspezifischen Merkmals, das heißt die Ausprägung der rhythmischen Aktivität im krankheitsspezifischen Frequenzbereich, nicht eineindeutig mit den krankheitsspezifischen Symptomen verbunden. Da der krankheitsspezifische Rhythmus Auswirkungen auf komplexe Nervennetzwerke im Gehirn hat, die typischerweise obendrein nicht einfachen linearen dynamischen Gesetzmäßigkeiten gehorchen, gelten keine eineindeutigen Relationen zwischen krankheitsspezifischem Rhythmus und Ausprägung der Symptome. Wenn zum Beispiel der krankheitsspezifische Rhythmus nicht hinreichend mit der biomechanisch vorgegebenen Eigenfrequenz einer Extremität übereinstimmt, ist der durch den krankheitsspezifischen Rhythmus bedingte Tremor deutlich geringer, als wenn der krankheitsspezifische Rhythmus in Resonanz mit der biomechanisch vorgegebenen Eigenfrequenz der Extremität übereinstimmt.

Die gemessene Aktivität befindet sich bei einer das Feedback-Signal erfassenden Lage des Sensors 3 in einem dem Fachmann bekannten Erfahrungsbereich. Der Wert der Ausprägung des krankheitsspezifischen Merkmals des über Sensor 3 gemessenen Feedback-Signals wird als Schwelle bezeichnet, bei dessen Überschreiten es typischerweise zum Auftreten von Symptomen, zum Beispiel des Tremors, kommt. Die Schwelle ist ein Parameter, der für die Ausführungsform des in Abschnitt 4.3. beschriebenen bedarfsgesteuerten Timings gewählt werden muss. Die erfindungsgemäße Vorrichtung umfasst daher Mittel zum Erkennen eines Schwellenwertes. Mit dem erfindungsgemäßen Verfahren des bedarfsgesteuerten Timings wird der Vorteil erreicht, dass die Wirksamkeit der erfindungsgemäßen Vorrichtung nicht kritisch von der Wahl der Schwelle abhängt, sondern bezüglich der Wahl der Schwelle eine große Fehlertoleranz gegeben ist, die beispielsweise in einem Bereich von bis zu 50 % der maximalen Ausprägung des krankheitsspezifischen Merkmals liegt. Die Wahl der Schwelle wird entweder intraoperativ oder vorzugsweise in den ersten Tagen nach der Operation durch Messung des Feedback-Signals über Sensor 3, mit Bestimmung der Ausprägung des krankheitsspezifischen Merkmals und Vergleich mit der Ausprägung der Symptome, z. B. der Stärke des Zitterns.

In einer weniger bevorzugten Ausführungsform des bedarfsgesteuerten Timings wird als Schwelle ein repräsentativer Wert, zum Beispiel der Mittelwert, eines Kollektivs von bei Patienten gemessenen Schwellenwerten genommen.

Erfindungsgemäß wird unter der Zielpopulation die unmittelbar durch eine implantierte Elektrode stimulierte Nervenzellpopulation verstanden.

Eine Zielpopulation wird durch eine in ihr oder nahe bei ihr implantierte Elektrode direkt stimuliert.

Die Nervenzellpopulation, welche krankhaft synchron aktiv ist, wird als zu desynchronisierendes Areal oder als zu desynchronisierende Nervenzellpopulation oder als zu desynchronisierende Neuronenpopulation bezeichnet. Letztere ist nicht an anatomische Grenzen gebunden. Vielmehr kann darunter auch mindestens eine Komponente, bestehend aus der Gruppe

- mindestens ein Teil von mindestens einem anatomischen Areal,
- mindestens ein vollständiges anatomisches Areal, verstanden werden.

Das zu desynchronisierende Areal kann entweder direkt oder indirekt stimuliert werden.

Direkte Stimulation über eine Stimulationselektrode 2: In diesem Fall befindet sich die Stimulationselektrode 2 in dem zu desynchronisierenden Areal. Diese Elektrode 2 beeinflusst dabei die Zielpopulation, welche sich in dem zu desynchronisierenden Areal befindet.

Indirekte Stimulation über eine Stimulationselektrode 2:

In diesem Fall wird das zu desynchronisierende Areal mittels Elektrode 2 nicht direkt stimuliert. Vielmehr wird über die Stimulationselektrode 2 eine Zielpopulation oder ein Faserbündel, welche mit dem zu desynchronisierenden Areal funktionell eng verbunden sind, stimuliert. Hierbei wird der Stimulationseffekt auf das zu desynchronisierende Areal vorzugsweise über anatomische Verbindungen fortgeleitet. Für die indirekte Stimulation soll als Oberbegriff für Zielpopulation und Faserbündel der Begriff Zielareal eingeführt werden. Von dem Begriff Zielareal sollen im Folgenden die mit dem zu desynchronisierenden Areal funktionell eng verbundene Neuronenpopulation und das verbindende Faserbündel verstanden werden.

Bei dem erfindungsgemäßen Stimulationsmechanismus wird innerhalb einer Periode der oszillatorischen Aktivität in der zu desynchronisierenden Neuronenpopulation über die einzelnen Elektroden 2 zu bestimmten, typischerweise unterschiedlichen Zeitpunkten stimuliert. Die Zeiträume zwischen diesen Einzelreizen werden als Bruchteile der Periode der oszillatorischen, zu desynchronisierenden Aktivität angegeben.

Ausführungsform für den Fall, dass alle Elektroden bzw. Kontaktstellen 14 in der zu desynchronisierenden Nervenzellpopulation positioniert sind:

Die N Elektroden bzw. Kontaktstellen 14 sollen vorzugsweise so angeordnet sein, dass mit jeder einzelnen Elektrode bzw. Kontaktstelle 14 ungefähr ein N-tel der zu desynchronisierenden Nervenzellpopulation stimuliert werden kann. Dies kann mit unterschiedlicher Anzahl der Elektroden bzw. Kontaktstellen 14 und mit unterschiedlicher geometrischer Anordnung der Elektroden bzw. Kontaktstellen 14 zueinander realisiert werden. Es kann beispielsweise eine beliebige, unsymmetrische Anordnung gewählt werden. Bevorzugt werden jedoch im Wesentlichen symmetrische Anordnungen, da bei diesen die stimulationsbedingte funktionelle Aufteilung in Subpopulationen mit dem geringsten Stromeintrag ermöglicht wird. Beispielhaft können die Endpunkte der Elektroden bzw. die Kontaktstellen 14 entlang der Elektroden projiziert, im Wesentlichen ein Quadrat ergeben. Es können beispielsweise auch 6 Elektroden bzw. Kontaktstellen 14 verwendet werden. Dabei liegen 4 vorzugsweise im Wesentlichen quadratisch angeordnet in einer Ebene, während die anderen beiden im Wesentlichen äquidistant senkrecht zu dieser Ebene liegen, wobei ihre Verbindungslinie im Wesentlichen die Rotationsachse der 4 quadratisch angeordneten Elektroden bildet. Zur Verwirklichung verschiedener geometrischer Anordnungen können die Elektroden bzw. Kontaktstellen 14 mindestens teilweise verschiedene Längen aufweisen.

Ausführungsform für den Fall, dass mindestens eine Elektrode 2 bzw. Kontaktstelle 14 nicht in der zu desynchronisiernden Nervenzellpopulation positioniert ist:

Bei dieser Stimulationsform wird in mindestens einem, von dem zu desynchronisierenden Areal, verschiedenen Zielareal stimuliert. Hierbei kann, wie oben beschrieben, die indirekte Stimulation durch Stimulation einer von der zu desynchronisierenden Nervenzellpopulation verschiedenen Neuronenpopulation und/oder durch Stimulation eines mit der zu desynchronisierenden Nervenzellpopulation verbundenen Faserbündels erfolgen. Dabei kann in einem Zielareal, beziehungsweise in dem zu desynchronisierenden Areal, entweder mindestens eine Elektrode 2 bzw. Kontaktstelle 14 oder eine oben beschriebene Mehrelektroden- bzw. mehr Kontaktstellen 14-Anordnung verwendet werden.

Der Wirkmechanismus der oben geschilderten erfindungsgemäßen Stimulationstechniken beruht darauf, dass über die zeitversetzte Reizung von Teilen der zu desynchronisierenden Neuronenpopulation lediglich eine Phasenverschiebung der kollektiven Oszillationen in den jeweiligen Teilpopulationen zueinander erzielt wird. Im Gegensatz zum Stand der Technik, das heißt insbesondere anders als bei dem in der Anmeldung DE 103 18 071 beschriebenen Verfahren ermöglichen es die erfindungsgemäßen Stimulationstechniken, einerseits mit minimaler Reizstärke und andererseits bei schwer betroffenen Patienten erstmals die Phasenverschiebung zwischen den kollektiven Oszillationen in den jeweiligen Teilpopulationen herzustellen. Die krankhafte Interaktion zwischen den Neuronen der Zielpopulation führt dann dazu, dass als Reaktion auf die Phasenverschiebung zwischen den Teilpopulationen die gesamte Zielpopulation transient durch einen desynchronisierten Zustand läuft. Diese durch die krankhaften Interaktionen zwischen den betroffenen Neuronen als Folge der gezielten erfindungsgemäßen Reizeinwirkung auftretende transiente Desynchronisation ist das Wirkprinzip der erfindungsgemäßen Vorrichtung. Eine repetitive Applikation von Reizen führt zu einer repetitiven Desynchronisation, so dass durch die erfindungsgemäße und oben beschriebene Regelung ein Zustand ausgeprägter Desynchronisation in der Zielpopulation aufrechterhalten wird.

Schon intraoperativ lässt sich beim Einführen der Tiefenelektrode der Stimulationserfolg sofort überprüfen. Hierdurch kann das Auffinden des geeigneten Zielpunkts deutlich verbessert werden. Für die bisherigen bedarfsgesteuerten Verfahren benötigt man eine Kalibrierung, welche pro Elektrode länger als 30 Minuten dauert. Das ist intraoperativ nicht durchführbar und dem (nicht narkotisierten) Patienten nicht zumutbar. Die neuen Stimulationsmethoden lassen sich auch bei neurologischen bzw. psychiatrischen Erkrankungen anwenden, bei denen pathologische Rhythmen stark schwankende Frequenzen aufweisen. Insbesondere lassen sich mit den neuen Methoden auch intermittente (d. h. kurzzeitig auftretende) Rhythmen desynchronisieren. Hieraus ergibt sich, dass die neuen Stimulationsmethoden bei weit mehr Erkrankungen zur Anwendung kommen können, vor allem auch bei den Epilepsien.

Mit der erfindungsgemäßen Vorrichtung können mit dem neuen Stimulationsverfahren folgende Krankheiten bzw. Symptome durch Desynchronisation geeigneter Hirnareale behandelt werden.

Bei allen neurologischen und psychiatrischen Erkrankungen, bei denen pathologische neuronale Synchronisation eine für die Ausprägung der krankheitsspezifischen Symptome eine relevante Rolle spielt, zum Beispiel: Morbus Parkinson, essentieller Tremor, Dystonie, Zwangserkrankungen, Tremor bei Multipler Sklerose, Tremor in Folge eines Schlaganfalls oder einer anderen, zum Beispiel tumorösen Gewebsschädigung, zum Beispiel im Bereich des Thalamus und/oder der Basalganglien, Choreoathetose und Epilepsie, wobei die Aufzählung nicht einschränkend sein soll.

Bei der zur Zeit verwendeten Standardmethode, der Hochfrequenz-Dauerstimulation, werden folgende Zielareale beispielhaft verwendet:

Bei Morbus Parkinson der Nucleus subthalamicus oder der Globus pallidum internum oder bei tremordominantem Morbus Parkinson der Thalamus, zum Beispiel der Nucleus ventralis intermedius thalami.
Bei essentiellem Tremor der Thalamus, zum Beispiel der Nucleus ventralis intermedius thalami.
Bei Dystonie und Choreoathetose der Globus pallidum internum.

Bei Epilepsie der Nucleus subthalamicus, der Hippocampus, die Substantia nigra, das Kleinhirn, thalamische Kerngebiete, zum Beispiel der Nucleus ventralis intermedius thalami, die Nuclei anteriores thalami, der Nucleus centromedianus thalami oder der Nucleus caudatus. Bei Tremor infolge von Multipler Sklerose der Nucleus ventralis intermedius thalami.

Bei Zwangserkrankungen die Capsula interna oder der Nucleus accumbens.

Bei der erfindungsgemäßen Vorrichtung können beispielsweise die für die jeweiligen Erkrankungen oben aufgeführten Zielareale gewählt werden. Weil bei der erfindungsgemäßen Vorrichtung entweder keine Kalibrierung notwendig ist oder die Kalibrierung sehr schnell durchgeführt werden kann, ergibt sich die Möglichkeit, im Rahmen der Elektrodenimplantation alternative Zielareale auszutesten, bei denen sich die desynchronisierende Wirkung der erfindungsgemäßen Vorrichtung noch besser entfalten lässt.

Die Erfindung umfasst ebenfalls eine Steuerung, welche die angegebene Funktionsweise der erfindungsgemäßen Vorrichtung steuert sowie die Verwendung der Vorrichtung und der Steuerung für die Behandlung der Krankheiten Morbus Parkinson, essentieller Tremor, Dystonie, Zwangserkrankungen, Choreoathetose, Tremor bei Multipler Sklerose, Tremor in Folge eines Schlaganfalls oder einer anderen, zum Beispiel tumorösen Gewebeschädigung, zum Beispiel im Bereich des Thalamus und/oder der Basalganglien, Epilepsie und Funktionsstörungen nach Schlaganfall.

Bei Funktionsstörungen nach Schlaganfall wird die den Schlaganfallsherd umgebende, in ihrer Funktion gestörte Zone stimuliert.

Die erfindungsgemäße Vorrichtung kann sowohl als Implantat zur dauerhaften Therapie der oben genannten neurologischen und psychiatrischen Erkrankungen als auch für die intraoperative Zielpunkt-Diagnostik, das heißt, die intraoperative Auffindung des optimalen Zielpunkts für die Elektrodenimplantation, verwendet werden.

**Patentansprüche**

1. Vorrichtung umfassend:

   - mindestens eine Elektrode (2) und/oder mindestens einen Chip zur Stimulation zerebraler Neuronen, welche eine pathologische oszillatorische Aktivität mit einer Periodendauer T aufweisen;
   - n Kontaktstellen (14), welche auf die mindestens eine Elektrode (2) und/oder auf den mindestens einen Chip verteilt sind, welche elektrische Reizsignale an das Gehirn abgeben; und
   - eine Steuerung (4), welche die Kontaktstellen (14) so ansteuert, dass sie niederfrequente Folgen von Hochfrequenz-pulszügen an das Gehirn abgeben,

   **dadurch gekennzeichnet, dass**

   - die Steuerung (4) die Kontaktstellen (14) derart ansteuert, dass sie eine niederfrequente Abfolge von Sequenzen von Hochfrequenz-Pulszügen an das Gehirn applizieren,
   - für jede Sequenz m Kontaktstellen (14) aus den n Kontaktstellen (14), über die in der jeweiligen Sequenz stimuliert wird, und die Reihenfolge, in der über die m ausgewählten Kontaktstellen (14) die Hochfrequenzpulszüge appliziert werden, ausgewählt werden, wobei $m \geq 2$ und $m < n$ gilt,

- innerhalb einer bestimmten Sequenz die zeitliche Verschiebung zwischen zwei aufeinander folgenden und über verschiedene der m ausgewählten Kontaktstellen (14) applizierten Hochfrequenzpulszügen T/m beträgt, und

- innerhalb der Abfolge der Sequenzen die m Kontaktstellen (14) und die Reihenfolge, in der über die m ausgewählten Kontaktstellen (14) die Hochfrequenzpulszüge appliziert werden, deterministisch und/oder stochastisch und/oder kombiniert deterministisch-stochastisch variiert werden.

2. Vorrichtung nach Anspruch 1, wobei die zeitlichen Abstände zwischen aufeinander folgenden Sequenzen deterministisch und/oder stochastisch und/oder kombiniert deterministisch-stochastisch variiert werden.

**Claims**

1. An apparatus comprising:

   - at least one electrode (2) and/or at least one chip for stimulating cerebral neurons which have a pathological oscillatory activity with a period duration T;
   - n contact points (14) which are distributed over the at least one electrode (2) and/or over the at least one chip and which emit electrical stimulation signals to the brain; and
   - a control (4) which controls the contact points (14) so that they emit low-frequency sequences of radio-frequency pulse trains to the brain,

   **characterised in that**

   - the control (4) controls the contact points (14) so that they apply a low-frequency succession of sequences of radio-frequency pulse trains to the brain;
   - for each sequence, m contact points (14) are selected from the n contact points (14) via which stimulation is carried out in the respective sequence, and the sequence in which the radio-frequency pulse trains are applied via the m selected contact points (14) is selected, where m≥ 2 and m < n;
   - the time displacement between two mutually following radio-frequency pulse trains applied via different ones of the m selected contact points (14) within a specific sequence amounts to T/m; and
   - within the succession of sequences, the m contact points (14) and the sequence in which the radio-frequency pulse trains are applied via the m selected contact points (14) are varied deterministically and/or stochastically and/or in a combined deterministic-stochastic manner.

2. An apparatus in accordance with claim 1, wherein the time intervals between mutually following sequences are varied deterministically and/or stochastically and/or in a combined deterministic-stochastic manner.

**Revendications**

1. Dispositif comprenant :

   - au moins une électrode (2) et/ou au moins une puce pour la stimulation de neurones cérébraux qui présentent une activité oscillatoire pathologique avec une durée de période T ;
   - n points de contact (14), qui sont répartis sur ladite au moins une électrode (2) et/ou sur ladite au moins une puce, qui délivrent des signaux d'excitation électriques au cerveau ; et
   - une commande (4) qui pilote les points de contact (14) de telle façon qu'ils délivrent au cerveau des successions à basse fréquence de salves d'impulsions à haute fréquence,

   **caractérisé en ce que**

   - la commande (4) pilote les points de contact (14) de telle façon qu'ils appliquent au cerveau une succession à basse fréquence de séquences de salves d'impulsions à haute fréquence,
   - pour chaque séquence, elle sélectionne parmi les n points de contact (14) m points de contact (14) via lesquels une stimulation a lieu dans la séquence respective et l'ordre dans lequel les trains d'impulsions à haute fréquence sont appliqués via les m points de contact sélectionnés (14), avec m ≥ 2 et m < n,
   - à l'intérieur d'une séquence déterminée, le décalage temporel entre deux trains d'impulsions à haute fréquence mutuellement successifs et appliqués via des points de contact (14) différents des m points de contact sélectionnés (14) est égal à T/m, et

- à l'intérieur de la succession de séquences, les m points de contact (14) et l'ordre dans laquelle les trains d'impulsions à haute fréquence sont appliqués via les m points de contact sélectionnés (14) sont variés de manière déterministe et/ou stochastique et/ou de manière combinée déterministe-stochastique.

2. Dispositif selon la revendication 1, dans lequel les écarts temporels entre des séquences mutuellement successives sont variés de manière déterministe et/ou stochastique et/ou de manière combinée déterministe-stochastique.

Fig.1

Fig. 2a

Fig. 2b

Fig:3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2004093981 A1

- DE 10318071

**In der Beschreibung aufgeführte Nicht-Patentliteratur**

- **VON P. TASS.** Detection of n:m Phase Locking from Noisy Data: Application to Magnetoencephalography. *Physical Review Letters,* 1998, vol. 81, 3291

- Phase resetting in Medicine and Biology. Stochastic Modelling and Data Analysis. **P.A. TASS.** Phase resetting in Medicine and Biology. Stochastic Modelling and Data Analysis. Springer Verlag, 1999